# EUROPEAN PATENT SPECIFICATION

(11) **EP 2 197 407 B1**
(45) Date of publication and mention of the grant of the patent: **01.01.2014**
(21) Application number: 08839503.3
(22) Date of filing: 17.10.2008
(51) Int. Cl.: A61G 12/00, G08B 21/04, A61B 5/11

(54) **Active floor mat to monitor sick person**
Aktive Bodenmatte zur Überwachung einer kranken Person
Tapis de sol actif pour surveiller une personne malade

(30) Priority: 19.10.2007 EP 07301481
(43) Date of publication of application: 23.06.2010
(73) Proprietor: INSERM - Institut National de la Santé et de la Recherche Médicale, 75013 Paris (FR)
(72) Inventor: STEENKESTE, François, F-11270 Laurac Le Grand (FR)
(74) Representative: Cabinet Plasseraud
(86) International application number: PCT/EP2008/064068
(87) International publication number: WO 2009/050285

(56) References cited:
- GB-A- 1 342 468
- US-A- 3 496 381
- US-A- 4 441 097
- US-A1- 2005 257 628
- US-A1- 2007 186 667

## Description

### Field of the invention

The invention relates to the field of active floor mat.

### Background of the invention

When a sick person is confided to bed due to his/her illness, there is a need for nurses and hospital personnel to be notified when the person falls from the bed or goes away from it. This need is particularly significant when the person is excited or gets mad as with Alzheimer illness, for instance.

To answer this need, different systems have been developed. For instance, the patent application JP2005185812 discloses a mat placed on the floor around the bed. The mat contains a pressure sensitive sensor such that, when a person falls down from the bed, the pressure sensitive sensor informs a contact site and a monitor displays an image of the bed taken by a monitoring camera.

The patent application JP6168391 discloses a floor mat installed on the floor of a lavatory room and divided into plural floor mat components. The lavatory room contains also a thermal ray type detector. The system judges that fall-down is generated in the lavatory room when more than two of the plural mat components send continuous human body pressure detection signals for more than a prescribed time and after recognizing that the thermal ray type detector transmits human body heat detection signals. Thus, there is less probability that more than a prescribed number of the plural mat components transmit the human body pressure detection signals even when hand luggage or the like is left alone on the floor of the room. Thus, the system hardly issues a false fall-down alarm.

Another type of a pressure sensing mat is disclosed in US 4, 441, 097.

However, these systems do not detect the leave of a person as the pressure variation on the mat generated by the feet of a person who sits on the bed, then stands up and then walks is much lower than the pressure variation generated by a fall. Further more, these systems need a mix of sensors, floor mat and camera or thermal ray detector, which are costly and often difficult to setup.

### Summary of the invention

It would advantageous to achieve a system able to detect the fall, and/or the leave, of a sick person from his/her bed, which is inexpensive and easy to setup.
To better address one or more concerns, a floor mat according to claim 1 comprises at least one presence sensor of a human body, characterized in that the sensor is adapted to detect a pressure variation on the floor mat and a body presence near the sensor, said sensor being further adapted to detect the pressure variation above a predetermined value and to detect a body presence when the pressure variation is below the predetermined value.

By combining two types of detection, i.e. pressure variation and presence detection, the floor mate has the advantage to be very sensitive whilst reducing false alarm.

In particular embodiment:
- each presence sensor comprises a capacitor comprising two conductive and flexible layers and a dielectric and resilient intermediate layer between the two conductive layers, said intermediate layer being deformed by a pressure exercised on the tile.
- each conductive layer for all the sensors is above 50% of the floor mat area.
- the sensor further comprises computing means adapted to detect a variation of the capacitor capacitance related to the deformation of the intermediate layer.
- the sensor further comprises computing means adapted to detect a charge transfer in the capacitor related to the body presence.
- the sensor further comprises communication means connected to a centralized control unit to send information of pressure variation and/or body presence with an identification code of the sensor.
- the sensor further comprises means to disconnect and/or calibrate it on request of the centralized control unit.
- the centralized control unit for receiving information of pressure variation and/or body presence from said tiles and for generating a control signal to alarm and/or supervision means.
- the centralized control unit is adapted to send an alarm signal when it received information of pressure variation and/or body presence from a number of contiguous tiles above a predetermined number.
- the centralized control unit is adapted to send to the supervision means information on the course of a person by analysis of the location of the tiles which send information of pressure variation and/or body presence as a time function.
- the centralized control unit is adapted to send to any tile of the floor mat a request to deactivate or to calibrate the sensor.
- it comprises a plurality of sensors organized in a regular network.
- the plurality of sensors is organized in a network of rows of capacitors, said mat further comprising a lane between the rows of capacitors and said lane comprising stripes of conductive material, each stripe being adapted to connect one capacitor electrode to input of a controller, each strip being in form of rafter such that any section of the lane contains substantially the same number of stripes

Advantageously, the mat is easy to manufacture as it does not require complex materials. By using a single capacitor in two detection modes, it reduces the cost of the mat whilst being sensitive to both a pressure and a presence.

Advantageously, the identification of each sensor increases the quality of the detector as it defines the area of the mat where the body is detected. For instance, a pressure variation just around a bed has a greater probability to indicate the fall of a sick person than a detection near the door of the room which may indicate the arrival of a nurse.

Advantageously, the centralized control unit may modify, or suppress the sensor detection, in part or in whole, which adapt the mat to its environment. For instance, the mat may be disabled during nursing or visit. Or some part of the mat may be calibrated to react to higher, or lower, pressure variation. The alarm detection may also be adapted by defining the number of sensors which must record a presence or a pressure variation.

These and other aspects of the invention will be apparent from and elucidated with reference to the embodiment described hereafter where:
- Figure 1 is a schematic and isometric view of a tile according to an embodiment of the invention;
- Figure 2 is a schematic view of a mat composed of tiles of Figure 1.
- Figure 3 is a perspective view of a mat according to a second embodiment; and
- Figure 4 is a top view of the mat of Figure 3.

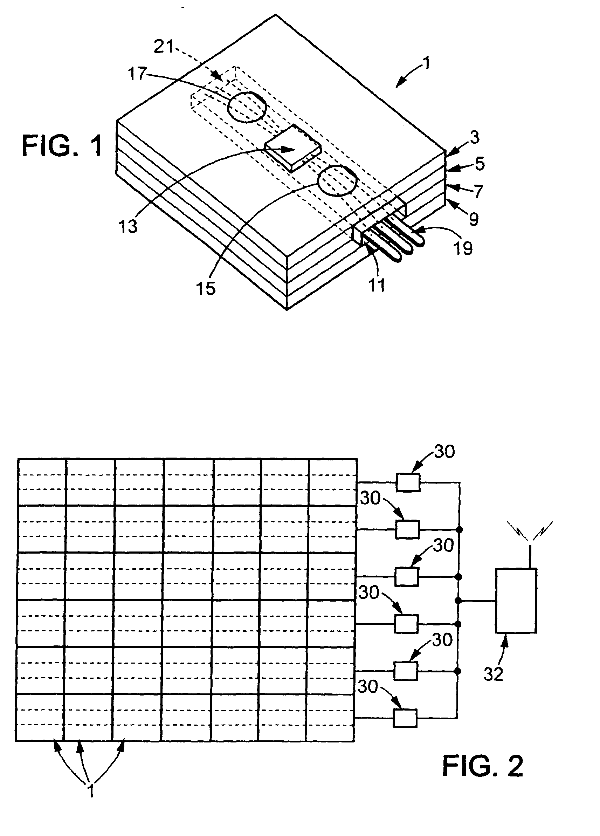

In reference to Figure 1, a floor tile 1 has a square shape of a standardized size of 50 x 50, or 30 x 30, square centimeter. It comprises four layers which are stacked up. The upper layer 3 is a wear and tear layer to protect the tile from person walk, dirt, etc and is made of plastic, for instance PVC, or any other suitable material. It protects an electrically conductive layer 5 which, with a dielectric layer 7 and a second electrically conductive layer 9 forms a capacitor. Optionally, a lower dielectric layer (not represented) may be used to protect the floor tile from the floor on which the tile 1 is put.

The dielectric layer 7 is composed of a flexible material such as foam.

So the dielectric layer 7 and the two conductive layers 5, 9 create a capacitor of which the capacitance is modified when a pressure is exercised on the tile 1.

As an example, with foam having a thickness of 3 millimeters and a density of 0.15, the capacitance varies from 5 to 16 % of a reference value for a weight pressing the tile of 4 to 75 kg.

The dielectric layer 7 comprises a void 11 in which a microcontroller 13 is fixed up. Two electrical cables 15, 17, such as ribbon cables, connect electrically the microcontroller 13 to two connectors 19, 21 disposed on two opposite sides of the tile 1. Preferably, the two connectors are of the male or female type each so that two contiguous tiles may be electrically connected together.

The microcontroller 13 is connected to the capacitor of the tile to form a presence sensor of the tile.

To detect the presence of a human body, the sensor comprises two working modes: a pressure variation mode and a proximity mode.

The pressure variation exercised by a part of a human body on the tile modifies the thickness of the dielectric layer 7 what generates a capacitance variation which is detected by the microcontroller 13.

The proximity mode is based on the charge transfer generates by the human body. When a part of a human body approaches to the tile, it generates in the upper conductive layer 5 a movement of the electric charges which is detected by the microcontroller 13.

These two working modes are complementary: to avoid false detection due to small electrical variations, the pressure variation detection is limited to variation above a predetermined value. However, this limitation inhibits the detection of small weight on the tile. For instance, an elderly person who falls from his/her bed to several tiles may generate only a small variation pressure on each tile as his/her weight is spread on different tiles.

At the opposite, a human body near the tile, at few millimeters, generates a charge transfer whatever the human body weight.

To form an active floor mat, a regular network of the floor tiles is formed, Figure 2.

On each row, the tiles are connected together electrically through the connectors up to a row controller 30. And the row controllers 30 are connected to a central controller 32.

The electrical connections between tiles create a bus to distribute the electrical power to the tiles and to transmit information from/to each tile to the row controller 30. Particularly, each tile is able to transmit presence information with an identification code of the tile so that the row controller and/or the central controller can know which tile is sending presence information.

Advantageously, the tile controller and the row controller are independent of the central controller for detection. Thus, there is no need for a scan of each row/tile from the central controller.

The row controller and/or the central controller use the bus to transmit to a tile requests to calibrate, to connect or to disconnect.

The central controller 32 centralizes all information coming from the tiles and generates control signal to alarm and/or supervision means.

By analyzing information coming from tiles, the central controller 32 is able to differentiate information generated by a standing person who generates a detection signal in 6 or 8 tiles at most and information generated by a falling person who generates a detection signal in around 20 tiles. The tile size, which is typically 30*30 square centimeters, may be modified to be adapted to the person to monitor. For instance, it may be advantageous to use smaller tiles to monitor children.

The mat may be used also to monitor and study the walking activity of a person. In this case, the central controller contains a map of the tiles to be able to correlate the presence information sent by tiles to their position in the mat. By adding the time information about the detection, the central controller is able to trace the itinerary of a person walking on the mat.

While the invention has been illustrated and described in details in the drawings and foregoing description, such illustration and description are to be considered illustrative or exemplary and not restrictive; the invention is not limited to the disclosed embodiment.

For instance, the tile size and form may vary depending on the type of use. As explained above, if it is necessary to monitor children of to have a precise map of the walking of a person, the mat will be composed of small, for instance 20*20 square centimeters, tiles. At the opposite, if the mat is only used to monitor the fall of an adult, large tiles, for instance 50*50 square centimeters tiles, may be used.

Although the square form has the advantage to define easily the mapping of every tile as a 2D matrix, other regular form may be used such as, for instance, the hexagonal form.

The size of the conductive layers compared to the total size of the tile may vary. However, the ratio conductive layer size / tile size is preferably above 50% to detect a pressure variation or a charge transfer on any part of the tile. To avoid that a contiguous tile generates a charge transfer, the conductive layer has advantageously a size which is less than the tile size so that the distance between the conductive layers of two contiguous tiles avoids an electrical coupling of the conductive layers.

To ease the management of the mat, as explained above, the central controller may send request to a specific tile to calibrate and/or (dis)connect.

The calibration may adjust different parameters of the sensor. For instance, it may modify the predetermined level of detection for pressure variation, the slow drift of the sensor and/or the number of consistent answers before the detection acknowledgement.

As an example of embodiment of these control requests, each request is composed of 5 bytes. The first byte is the control to be executed, the second byte is the row in the mat matrix, the third byte is the tile number in the row, the fourth byte is the parameter value to be modified and the fifth byte is a CRC to validate the transmission of the request. As an acknowledgement, the tile microcontroller sends a five byte answer in which the first byte is the ASCII value of 'V' as Validate, the second, third and fourth bytes are not modified and the fifth byte is a new CRC taking into account the modification of the first byte.

In the described embodiment, each tile has a microcontroller. This embodiment has the advantage to reduce the data flow on the mat bus. However, to reduce cost, it is possible to have only a row controller to control the tiles and to reduce the electronics of each tile to the sensing of pressure variation and charge transfer.

It is also possible to integrate the tiles into the mat, i.e to make a one-piece mat containing a 2D matrix of sensors. Although, this embodiment has the disadvantage to define the mat dimension at the manufacturing step, it has the advantage to reduce the cost of the mat by optimizing the sensor connection (there is no need for connectors for each sensor) and defining an electronic structure optimized for the mat, for instance, by sharing a microcontroller with a plurality of capacitors.

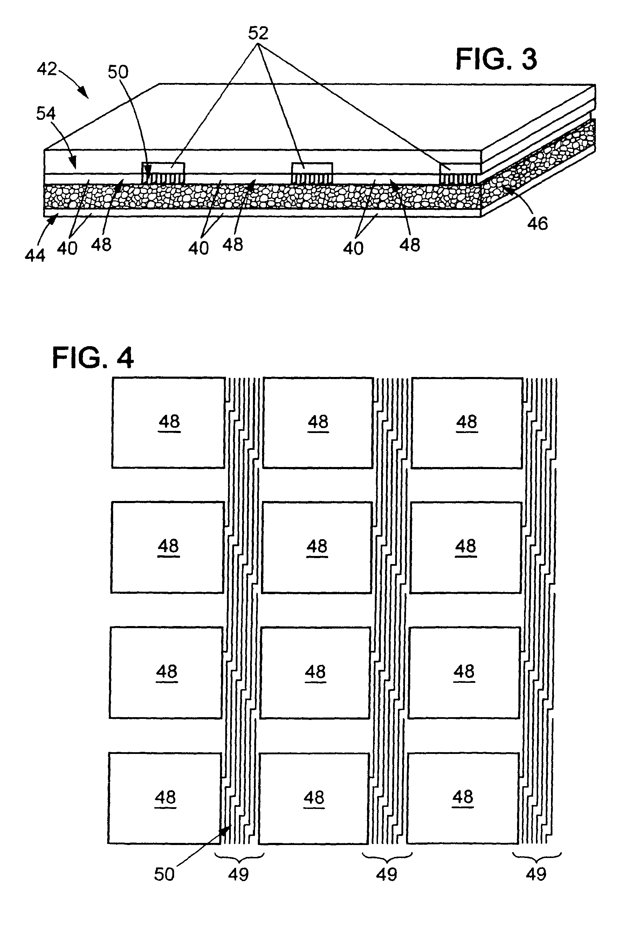

An example of these embodiments is disclosed in the relation with Figures 3 and 4, in which all control and sensing electronics are merged into the row controller.

Capacitors 40 are forming a network on the active floor mate 42. For instance, the mat 42 comprises 3 rows of 4 capacitors each.

A continuous conductor sheet 44 is forming a first electrode of the capacitors 42. The sheet 44 is connected to ground.

A foam 46, such as a polyurethane foam, is disposed on top of the conductor sheet 44 to form the dielectric medium of the capacitors.

On top of the foam 46, squares 48 of conductor material form the second electrodes of the capacitors.

Between each row of conductor squares, a lane 49 contains strips 50 of conductive materials to connect each square, i.e each capacitor, to a microcontroller input (not represented).

In order to protect these strips from parasitic field, they are covered by a shield 52 such as, for instance, an aluminum sheet connected to ground.

A protective layer 54 covers the set of capacitors and protect them from person walk, dust, etc.

The conductive strips 50 are in form of rafter. Therefore, if, for the sake of explanation, the lane 48 is adapted to contain 16 strips 50, for instance, it means that the mat may be manufactured on a roll to be cut in place at the right size of the place to protect with a maximum of 16 capacitors per row.

With such a mat, the capacitors have a low capacitance of less than 300pF, typically 200pF, to obtain a good compromise between the pressure variation detection and the charge transfer detection.

Advantageously, with the transfer of electronics into the row controller the mat thickness is reduced as there is no more need to reserve space for electronics. The thickness reduction allows for an easier manufacturing process and thus a cost reduction.

Each row is independent of the other rows. Therefore the mat can be adapted to any irregular surface. For instance, in a bedroom, it can be chosen to not place the active mat under the furnitures. Consequently, each row may have a different length and a different number of tiles/capacitors, the row controller being parameterized to the correct number of capacitors to control.

It is not even necessary to align capacitors in columns: the only constraint is that the floor plan of the central controller reproduces correctly the position of each tile in the room.

Another embodiment consists of tiles containing a plurality of sensors. For instance, a rectangular tile with two square sensors or a square tile with four square sensors may be manufactured. This embodiment combines some advantages of the single-piece mat in term of cost and optimization and some advantages of the single sensor tiles in term of versatility.

Other variations to the disclosed embodiments can be understood and effected by those skilled on the art in practicing the claimed invention, from a study of the drawings, the disclosure and the appended claims. In the claims, the word "comprising" does not exclude other elements and the indefinite article "a" or "an" does not exclude a plurality.

## Claims

1. Floor mat comprising at least one presence sensor (5, 7, 9, 13) of a human body, **characterized in that** the sensor is adapted to detect a pressure variation on the floor mat and a body presence near the sensor, said sensor being further adapted to detect the pressure variation above a predetermined value and to detect a body presence when the pressure variation is below the predetermined value.

2. Floor mat according to claim 1, **characterized in that** each presence sensor comprises a capacitor comprising two conductive and flexible layers (5, 9) and a dielectric and resilient intermediate layer (7) between the two conductive layers, said intermediate layer being deformed by a pressure exercised on the tile.

3. Floor mat according to claim 2, **characterized in that** the total of each conductive layer for all the sensors is above 50% of the floor mat area.

4. Floor mat according to claim 2 or 3, **characterizing in that** the capacitor has a capacitance of less than 300pF when no pressure is applied

5. Floor mat according to claim 2 or 3, **characterized in that** the sensor further comprises computing means (13) adapted to detect a variation of the capacitor capacitance related to the deformation of the intermediate layer.

6. Floor mat according to one of any preceding claims, **characterized in that** the sensor further comprises communication means connected to a centralized control unit (32) to send information of pressure variation and/or body presence with an identification code of the sensor.

7. Floor mat according to claim 6, **characterized in that** the sensor further comprises means to disconnect and/or calibrate it on request of the centralized control unit.

8. Floor mat according to claim 6 or 7, **characterized in that** it further comprises the centralized control unit for receiving information of pressure variation and/or body presence from said tiles and for generating a control signal to alarm and/or supervision means.

9. Floor mat according to claim 8, **characterized in that** the centralized control unit is adapted to send an alarm signal when it received information of pressure variation and/or body presence from a number of contiguous tiles above a predetermined number.

10. Floor mat according to claim 8 or 9, **characterized in that** the centralized control unit is adapted to send to the supervision means information on the course of a person by analysis of the location of the tiles which send information of pressure variation and/or body presence as a time function.

11. Floor mat according to claim 8, 9 or 10, **characterized in that** the centralized control unit is adapted to send to any tile of the floor mat a request to deactivate or to calibrate the sensor.

12. Floor mat according to any preceding claims, **characterized in that** it comprises a plurality of sensors organized in a regular network.

13. Floor mat according to claim 12, **characterized in that** the plurality of sensors is organized in a network of rows of capacitors, said mat further comprising a lane between the rows of capacitors and said lane comprising stripes of conductive material, each stripe being adapted to connect one capacitor electrode to input of a controller, each strip being in form of rafter such that any section of the lane contains substantially the same number of stripes.

## Patentansprüche

1. Bodenmatte, umfassend wenigstens einen Anwesenheitssensor (5, 7, 9, 13) eines menschlichen Körpers, **dadurch gekennzeichnet, dass** der Sensor dazu eingerichtet ist, eine Druckveränderung auf der Bodenmatte und eine Körperanwesenheit nahe des Sensors zu detektieren, wobei der Sensor ferner dazu eingerichtet ist, die Druckveränderung über einem vorbestimmten Wert zu detektieren und eine Körperanwesenheit zu detektieren, wenn die Druckveränderung unter dem vorbestimmten Wert liegt.

2. Bodenmatte nach Anspruch 1, **dadurch gekennzeichnet, dass** jeder Anwesenheitssensor einen Kondensator umfasst, welcher zwei leitende und flexible Schichten (5, 9) und eine dielektrische und elastische Zwischenschicht (7) zwischen den beiden leitenden Schichten umfasst, wobei die Zwischenschicht durch einen auf die Kachel ausgeübten Druck deformiert wird.

3. Bodenmatte nach Anspruch 2, **dadurch gekennzeichnet, dass** die Gesamtheit einer jeden leitenden Schicht für sämtliche Sensoren über 50% der Bodenmattenfläche liegt.

4. Bodenmatte nach Anspruch 2 oder 3, **dadurch gekennzeichnet, dass** der Kondensator eine Kapazität von weniger als 300pF aufweist, wenn kein Druck ausgeübt wird.

5. Bodenmatte nach Anspruch 2 oder 3, **dadurch gekennzeichnet, dass** der Sensor ferner Berechnungsmittel (13) umfasst, welche dazu eingerichtet sind, eine mit der Deformation der Zwischenschicht in Zusammenhang stehende Veränderung der Kondensatorkapazität zu detektieren.

6. Bodenmatte nach einem der vorhergehenden Ansprüche, **dadurch gekennzeichnet, dass** der Sensor ferner Kommunikationsmittel umfasst, welche mit einer zentralen Steuerungs-/Regelungseinheit (32) verbunden sind, um Informationen einer Druckveränderung oder/und einer Körperanwesenheit mit einem Identifizierungscode des Sensors zu senden.

7. Bodenmatte nach Anspruch 6, **dadurch gekennzeichnet, dass** der Sensor ferner Mittel umfasst, um ihn zu trennen oder/und zu kalibrieren auf Anfrage der zentralen Steuerungs-/Regelungseinheit.

8. Bodenmatte nach Anspruch 6 oder 7, **dadurch gekennzeichnet, dass** sie ferner die zentrale Steuerungs-/Regelungseinheit umfasst, um Informationen einer Druckveränderung oder/und Körperanwesenheit von den Kacheln zu empfangen und um ein Steuerungs-/Regelungssignal für Alarm- oder/und Überwachungsmittel zu erzeugen.

9. Bodenmatte nach Anspruch 8, **dadurch gekennzeichnet, dass** die zentrale Steuerungs-/Regelungseinheit dazu eingerichtet ist, ein Alarmsignal zu senden, wenn sie Informationen einer Druckveränderung oder/und Körperanwesenheit von einer Anzahl von zusammenhängenden Kacheln empfängt, die größer als eine vorbestimmte Anzahl ist.

10. Bodenmatte nach Anspruch 8 oder 9, **dadurch gekennzeichnet, dass** die zentrale Steuerungs-/Regelungseinheit dazu eingerichtet ist, den Überwachungsmitteln Informationen über den Weg einer Person anhand einer Analyse der Position der Kacheln zu senden, welche Informationen einer Druckveränderung oder/und Körperanwesenheit als eine Zeitfunktion senden.

11. Bodenmatte nach Anspruch 8, 9 oder 10, **dadurch gekennzeichnet, dass** die zentrale Steuerungs-/Regelungseinheit dazu eingerichtet ist, einer Kachel der Bodenmatte eine Anfrage zum Deaktivieren oder zum Kalibrieren des Sensors zu senden.

12. Bodenmatte nach einem der vorhergehenden Ansprüche, **dadurch gekennzeichnet, dass** sie eine Mehrzahl von Sensoren umfasst, welche in einem regelmäßigen Netzwerk organisiert ist.

13. Bodenmatte nach Anspruch 12, **dadurch gekennzeichnet, dass** die Mehrzahl von Sensoren in einem Netzwerk aus Reihen von Kondensatoren organisiert ist, wobei die Matte ferner eine Bahn zwischen den Reihen von Kondensatoren umfasst und die Bahn Streifen leitfähigen Materials umfasst, wobei jeder Streifen dazu eingerichtet ist, eine Kondensatorelektrode mit einem Eingang eines Controllers zu verbinden, wobei jeder Streifen die Form eines Sparrens aufweist, so dass jeder Abschnitt der Bahn im Wesentlichen die gleiche Anzahl von Streifen beinhaltet.

## Revendications

1. Tapis de sol comprenant au moins un détecteur de présence (5, 7, 9, 13) d'un corps humain, **caractérisé en ce que** le détecteur est adapté pour détecter une variation de pression sur le tapis de sol et la présence d'un corps à proximité du détecteur, ledit détecteur étant en outre adapté pour détecter la variation de pression au-dessus d'une valeur prédéterminée et pour détecter la présence d'un corps lorsque la variation de pression est inférieure à la valeur prédéterminée.

2. Tapis de sol selon la revendication 1, **caractérisé en ce que** chaque détecteur de présence comprend un condensateur comprenant deux couches conductrices et flexibles (5, 9) et une couche intermédiaire diélectrique et résiliente (7) entre les deux couches conductrices, ladite couche intermédiaire étant déformée par une pression exercée sur le carreau.

3. Tapis de sol selon la revendication 2, **caractérisé en ce que** le total de chaque couche conductrice pour tous les détecteurs est supérieur à 50 % de la surface du tapis de sol.

4. Tapis de sol selon la revendication 2 ou 3, **caractérisé en ce que** le condensateur a une capacité inférieure à 300 pF lorsqu'aucune pression n'est appliquée.

5. Tapis de sol selon la revendication 2 ou 3, **caractérisé en ce que** le détecteur comprend en outre des moyens de calcul (13) adaptés pour détecter une variation de la capacité du condensateur associée à la déformation de la couche intermédiaire.

6. Tapis de sol selon l'une quelconque des revendications précédentes, **caractérisé en ce que** le détecteur comprend en outre des moyens de communication raccordés à une unité de commande centralisée (32) pour envoyer des informations de variation de pression et/ou de présence d'un corps avec un code d'identification du détecteur.

7. Tapis de sol selon la revendication 6, **caractérisé en ce que** le détecteur comprend en outre des moyens pour sa déconnexion et/ou son calibrage à la demande de l'unité de commande centralisée.

8. Tapis de sol selon la revendication 6 ou 7, **caractérisé en ce qu'**il comprend en outre l'unité de commande centralisée pour recevoir des informations sur la variation de pression et/ou la présence d'un corps desdits carreaux pour générer un signal de commande d'une alarme et/ou des moyens de surveillance.

9. Tapis de sol selon la revendication 8, **caractérisé en ce que** l'unité de commande centralisée est adaptée pour envoyer un signal d'alarme lorsqu'il a reçu des informations sur la variation de pression et/ou la présence d'un corps d'un certain nombre de carreaux contigus, supérieur à un nombre prédéterminé.

10. Tapis de sol selon la revendication 8 ou 9, **caractérisé en ce que** l'unité de commande centralisée est adaptée pour envoyer aux moyens de surveillance des informations sur le parcours d'une personne par analyse de l'emplacement des carreaux qui envoient des informations sur la variation de pression et/ou la présence d'un corps, en fonction du temps.

11. Tapis de sol selon la revendication 8, 9 ou 10, **caractérisé en ce que** l'unité de commande centralisée est adaptée pour envoyer à un carreau quelconque du tapis de sol une demande pour désactiver ou calibrer le détecteur.

12. Tapis de sol selon l'une quelconque des revendications précédentes, **caractérisé en ce qu'**il comprend une pluralité de détecteurs organisés en un réseau régulier.

13. Tapis de sol selon la revendication 12, **caractérisé en ce que** la pluralité de détecteurs est organisée en un réseau de rangées de condensateurs, ledit tapis comprenant en outre une voie entre les rangées de condensateurs, et ladite voie comprenant des bandes de matériau conducteur, chaque bande étant adaptée pour raccorder une électrode de condensateur à l'entrée d'un dispositif de commande, chaque bande étant sous la forme de chevrons de telle sorte que chaque partie de la voie contienne sensiblement le même nombre de bandes.
